**Europäisches Patentamt**

**European Patent Office**

**Office européen des brevets**

(19)

(11) Veröffentlichungsnummer: **0 023 608**
**A1**

# EUROPÄISCHE PATENTANMELDUNG

(12)

(21) Anmeldenummer: **80104016.3**

(22) Anmeldetag: **11.07.80**

(51) Int. Cl.³: **A 61 F 1/00**, A 61 B 17/18, A 61 C 8/00, A 61 K 6/00

(30) Priorität: **11.07.79 DE 2928007**

(43) Veröffentlichungstag der Anmeldung: **11.02.81**
**Patentblatt 81/6**

(84) Benannte Vertragsstaaten: **AT CH DE FR GB IT LI SE**

(71) Anmelder: **Riess, Guido, Dr. med. dent., Marienplatz 7, D-8100 Garmisch-Partenkirchen (DE)**

(72) Erfinder: **Riess, Guido, Dr. med. dent., Marienplatz 7, D-8100 Garmisch-Partenkirchen (DE)**
Erfinder: **Geiger, Albert, Breitenau, D-8100 Garmisch-Partenkirchen (DE)**

(74) Vertreter: **Körber, Wolfhart, Dr. et al, Patentanwälte Dipl.Ing.H.Mitscherlich, Dipl.Ing.K.Gunschmann, Dr.rer.nat.W.Körber, Dipl.Ing.J.Schmidt-Evers Steinsdorfstrasse 10, D-8000 München 22 (DE)**

(54) Knochen-Implantatkörper für Prothesen und Knochenverbindungsstücke sowie Verfahren zu seiner Herstellung.

(57) Es wird ein Knochen-Implantatkörper für Knochen- und Gelenk-, sowie dentale Prothesen, Knochenverbindungsstücke wie Knochenschrauben und -schienen, bestehend aus einem mechanisch stabilen und mit Knochengewebe verträglichen (biokompatiblen) Trägerwerkstoff und darin eingelagerter bioreaktiver Calciumphosphatkeramik, insbesondere aus Tri- und Tetracalciumphosphat beschrieben.

Um einen solchen Implantatkörper mit dauerhafter Biokompatibilität in allen gewünschten Formen mit genügender Genauigkeit herstellen zu können, ist der Trägerwerkstoff ein biokompatibles Metall, wie Titan, Gold, Platin, rostfreier Stahl oder ein ähnliches schadlos ohne Bildung von Zwischenreaktionsprodukten mit Calciumphosphatkeramik verbundfähiges Sintermetall. Dabei besteht die Oberflächenschicht zweckmässig aus mehreren verschieden hoch gesinterten oder verschieden hoch gepressten, übereinander gelagerten Calciumphosphat-Schichten.

Ein Herstellungsverfahren für den Implantatkörper besteht darin, dass ein Pulver des biokompatiblen Metalls mit feindispers bis stückig vorliegender Calciumphosphatkeramik vermischt und durch Sintern unter hohem Druck miteinander verbunden werden. Dabei wird zweckmässig die Oberflächenschicht aus reinem Calciumphosphat durch einen oder mehrere weitere Druck-Sintervorgänge auf den Implantatkörper aufgepresst.

Knochen-Implantatkörper für Prothesen und Knochenverbindungsstücke sowie Verfahren zu seiner Herstellung

Die Erfindung bezieht sich auf einen Knochen-Implantatkörper für Knochen- und Gelenk-, sowie dentale Prothesen, Knochenverbindungsstücke wie Knochenschrauben und -schienen, bestehend aus einem mechanisch stabilen und mit Knochengewebe verträglichen (biokompatiblen) Trägerwerkstoff und darin eingelagerter bioaktiver Calciumphosphatkeramik, inbesondere aus Tri- und Tetracalciumphosphat, sowie auf/ein Verfahren zur Herstellung eines solchen Implantatkörpers.

Bisher bekannte und meist verwendete Implantate bestehen aus einem in den Knochen einzusetzenden Verankerungsteil aus Metall und sind in Form einer Platte, Nadel, Schraube oder dergleichen ausgebildet und beruhen auf einer rein mechanischen Verzahnung mit dem Knochen, um eine Verankerung der Prothese am Knochen zu erreichen. Inzwischen wurde erkannt , dass mehrere werkstofftechnische Forderungen gleichzeitig erfüllt sein müssen, um eine dauernde stabile Implantierung zu erreichen. Die verwendeten Werkstoffe müssen mit dem Knochen biokombatibel sein und die Formgebung der

0023608

Implantate und die mechanischen Eigenschaften der Werkstoffe müssen eine biologisch richtige Belastung und Krafteinleitung gewährleisten, da der Knochen andernfalls durch Abbau und schließlich durch Lockerung des Implantates reagiert. Es wurde weiter erkannt, dass das Implantat in allen Bereichen mit dem Knochen eine direkte, dauerhafte, knöcherne Verbindung aufweisen muß und nicht durch eine Bindegewebsmembran vom Knochen abgekapselt werden darf.

Dazu sind in neuerer Zeit bioaktive Werkstoffe bekanntgeworden, die eine bindegewebsfreie Verwachsung des Knochens mit der Werkstoffoberfläche des Verankerungsteiles bewirken. Bei solchen Werkstoffen handelt es sich z.B. um Calciumphosphate bestimmter Zusammensetzung, bei denen eine direkte bindegewebsfreie Verwachsung des Knochens mit dem Werkstoff eintritt (Köster, "Experimenteller Knochenersatz durch resorbierbare Calciumphosphatkeramik", Langenbecks Archiv für Chirurgie 341,77-86 (1976). Diese Calciumphosphate sind im biologischen Milieu abbaubar, d.h. sie werden von den an der Knochenumbildung beteiligten Zellen absorbiert und erfüllen somit die gestellte biochemische Grundforderung, kommen aber als alleiniger Werkstoff bei einer auf Dauer implantierten Prothese mangels genügender Eigenfestigkeit und mangels dauernder Verankerung zwischen Werkstoff des Verankerungsteils und des Knochens wegen der gegebenen Resorbierbarkeit nicht in Betracht.

Um eine dauerhafte Verankerung von hochbelasteten Implantaten zu schaffen, bei der es zu einer wirklich dauerhaften Verbindung zwischen der Prothese und dem Gewebe kommt, ist es bekannt geworden, (DE-OS 26 20 907) die Prothesenverankerung als Prothesenschaftbeschichtung aus im Körpermilieu mechanisch und chemisch stabilen Kunststoff auszubilden und die keramischen Calciumphosphate in partikulärer Form bestimmter Partikelgröße im Durchmesser in den Kunststoff derart einzulagern, das bei der Resorption des keramischen Anteils ein durchgängig poröses Gefüge aus Kunststoff entsteht, auf dessen inneren Porenoberflächen bioaktivierende Reste der Keramik zurückbleiben.

Nach einem anderen Vorschlag für eine implantierbare Zahnwurzel (DE-OS 27 33 394) besteht diese im wesentlichen aus einer mit menschlichem Zellgewebe verträglichen, biostabilen Polymermatrix, in die resorbierbare bioreaktive Calciumphosphate in feindisperser Form eingelagert sind, die von einer dünnen porösen Schicht nicht resorbierbarer Calciumphosphate umgeben sind und in die ein Kern als Verbindungsstück zur Anbringung einer dentalen Suprastruktur eingefügt ist.

Gegen die Verwendung von Kunststoffen in Form einer Polymermatrix als Trägerkörper für die keramischen Calciumphosphate bestehen gewisse Bedenken, obwohl sie sich derzeit in der Praxis noch recht gut bewähren. Polymere Kunststoffe enthalten oft auch Monomere und andere Schadstoffe, die bei Implantaten nach entsprechender Alterung zu Austauschvorgängen mit dem Gewebe führen können. Auch erlaubt ein Kunststoffträgerkörper keine genügend feine Formgebung und mechanische Bearbeitung, um ein mit einem Kunststoffträgerkörper ausgestattetes Implantat für die verschiedensten Zwecke wie Knochen- und Gelenkprothesen oder auch für Knochenverbindungsstücke wie Schrauben, Schienen und dergleichen ausbilden zu können.

0023608

Der Erfindung liegt die Aufgabe zugrunde, einen Knochen-Implantatkörper und insbesondere einen Verbundwerkstoff für derartige Implantatkörper zu schaffen, bezüglich dessen dauernder Biokompa-tibilität keine Bedenken bestehen und der in allen gewünschten Formen mit genügender Genauigkeit hergestellt und mechanisch bearbeitet werden kann.

Zur Lösung dieser Aufgabe wird ein Knochenimplantatkörper der eingangs genannten Art vorgeschlagen, der dadurch gekennzeichnet ist, daß der Trägerwerkstoff ein biokompatibles Metall, wie Titan, Tantal, Niob oder ein ähnliches, schadlos ohne Bildung von Zwischenreaktionsprodukten mit Calciumphosphatkeramik verbundfähiges Sintermetall ist. Voraussetzung für die verwendeten Metalle ist eine nachgewiesene Biokompatibilität und Korrosionsarmut im Knochengewebe. Als momentan diese Ansprüche am meisten erfülllendes Metall kommt das Titan in Frage. Es wurde gefunden, dass Calciumphosphatkeramiken, die feindispers bis stückig als Pulver vorliegen mit Titanpulver etwa gleicher Korngröße durch Press- und Sinterverfahren zu einem Verbundwerkstoff vereinigt werden können, der die physikalischen und chemischen Vorteile der Summe der einzelnen Werkstoffe aufweist. Erreicht sind die physikalischen Vorteile des Metalles, hier Titan, mit seiner Korrosionsarmut, physikalischen Stabilität, relativ hoher Biokompatiblität und erreicht sind die biochemischen Vorteile der Calciumphosphatkeramik, insbesondere der Tricalciumphosphate, in ihrer bioaktiven Resorbierbarkeit. Zwar ist dieses Prinzip der Kombinationswirkung bei den bisher verwendeten Kunststoff-Verbundwerkstoffen aus Calciumphosphatkeramiken mit Polymeren bekannt. Jedoch bestehen bezüglich der Polymerkunststoffe die eingangs genannten Bedenken.

0023608

Bisher ist es nicht für möglich gehalten worden, auch Metalle mit Calciumphosphatkeramiken zu einem einheitlichen mechanisch stabilen Körper zu verbinden unter Beibehaltung der bioaktiven Eigenschaften der Calciumphosphate. Der Vorteil der Anwendung von Metallen als Trägerwerkstoff ist die höhere und berechenbarere langfristige Stabilität im Knochengewebe. Außerdem hat sich das Titan in nahezu 20-jähriger Erprobung im Knochengewebe als das bestverträglichste Metall gezeigt. Der Korrosionsgehalt ist relativ gering und somit die biochemische Abstoßungspotenz klein.

Ein Verfahren zur Herstellung eines erfindungsgemäßen Implantatkörpers ist dadurch gekennzeichnet, daß ein Pulver des biokompatiblen Metalles mit feindispers bis stückig vorliegender Calciumphosphatkeramik vermischt und durch Sintern unter hohem Druck miteinander verbunden werden. Zweckmäßig werden Metallpulver und Calciumphosphatkeramik in etwa gleicher Korngröße in etwa gleichem Volumenverhältnis (Raumerfüllung) miteinander vermischt und gesintert. Die Sintertemperatur liegt im Bereich von über $1500^{O}$ K (Grad Kelvin) bis $2300^{O}$ K je nach angestrebter Sinterdichte des Calciumphosphatträgerkörpers. Je niedriger die Sintertemperatur gehalten werden kann, um so besser ist die Resorbierbarkeit der Calciumphosphatkeramik. Der gleichzeitig mit dem Sintervorgang aufgebrachte hohe Druck liegt bei $10^{6}$ kPa (10.000 bar). Im zusammengesinterten Implantatkörper ist im Inneren eine schlechtere Resorbierbarkeit dafür aber eine größe Formstabilität erwünscht. Durch entsprechende Führung des Sinterverfahrens bzw. Einbringung der Komponenten ist es möglich, die Calciumphosphatkeramik im wesentlichen im Bereich der Oberfläche des Metall- oder Sintermetallkörpers anzuordnen bzw. anzureichern.

In besonders zweckmäßiger Ausgestaltung der Erfindung weist der Implantatkörper zumindest an seinen der Knochenfläche im Implantatraum innerhalb des Knochens zugewandten Seiten eine vollständig aus Calciumphosphat, insbesondere Tricalciumphosphat bestehende Oberflächenschicht auf. Aufgrund vorliegender experimenteller und klinischer Erfahrungen sollte die reine Calciumphosphat-Oberflächenschicht eine Stärke von 0,1 bis 0,5 mm haben. Erfindungsgemäß wird die Oberflächenschicht aus reinem Calciumphosphat durch einen weiteren Druck-Sintervorgang auf den Implantatkörper aufgepreßt. Die aufgesinterte Tricalciumphosphat-Oberflächenschicht ist mit den im Verbundwerkstoff Metall-Calciumphosphat enthaltenen Calciumphosphatpartikeln homogen verbunden – während im Verbundwerkstoff selbst mehr eine mechanische Verbindung zwischen Metall und Calciumphosphat vorliegt – so daß die Tricalciumphosphat-Oberflächenschicht in fester Verbundbildung mit dem Trägerkörper vorliegt. Bisher konnten Keramikbeschichtungen nur in einem Aufbrennverfahren durch starke Adhäsion mittels Oxidschichten und Haftvermittlern auf Metall aufgebracht werden. Durch den erfindungsgemäßen Verbundwerkstoff liegen in einem Metallträger gebundene Calciumphosphatbereiche vor, an welchen die Oberflächenschicht aus reinem Calciumphosphat in idealer Weise unmittelbar und fest verankert werden kann. Für das bindegewebslose Verwachsen des Knochengewebes war eine solche reine Calciumphosphatkontaktschicht anzustreben. Bisher war es nur möglich, Calciumphosphatbeschichtungen auf polymeren Verbundwerkstoffen oder auf Glaskeramik aufzubringen.

In weiterer Ausbildung der Erfindung ist die Oberflächenschicht durch degraduiert aufgebrachte Sinterdrücke im Bereich ihrer äußeren Oberfläche resorbierbar und ist mit zunehmender Tiefe zum Implantatkörper hin immer weniger bis nicht resorbierbar. Die Herstellung einer solchen Oberflächenschicht erfolgt erfindungsgemäss durch Aufpressen der Oberflächenschicht durch aufeinanderfolgende degraduierte Druck-Sintervorgänge, durch welche die Oberflächenschicht im Bereich ihrer äußeren Oberfläche durch relativ niedrige Sintertemperaturen resorbierbar bleibt und mit zunehmender Tiefe zum Implantatlager hin durch höhere Sintertemperaturen immer weniger bis nicht resorbierbar wird.

In weiterer besonders zweckmäßiger Ausbildung der Erfindung besteht die Oberflächenschicht aus mehreren verschieden hoch gesinterten oder verschieden hoch gepreßten, übereinander gelagerten Calciumphosphat-Schichten, wobei die äußere Oberschicht aus reinem niedrig gesintertem, gut resorbierbarem Tricalciumphosphat, eine mittlere, das Knochenwachstum bremsende Schicht aus hochgesintertem, schwer bis nicht resorbierbarem Tricalciumphosphat und eine innere Trägerschicht aus nicht resorbierbarem, hochgesintertem Calciumphosphat bestehen. Dabei kann zweckmässig die inntere Trägerschicht auch aus nicht resorbierbarem Tetracalciumphosphat, gegebenenfalls in Mischung mit hochgesintertem Tricalciumphosphat bestehen. Die Herstellung dieser einzelnen übereinandergelagerten Calciumphosphat-Schichten der Oberflächenschicht erfolgt erfindungsgemäß durch Aufpressen in aufeinanderfolgenden Druck-Sintervorgängen, wobei die innere Trägerschicht und die Bremsschicht bei vergleichsweise hohen Sintertemperaturen und/oder Drücken und die äußere Oberschicht bei vergleichsweise niedriger Sintertemperatur aufgepreßt werden.

Der letztgenannten Ausbildung der Erfindung liegt das Bestreben zugrunde, unabhängig von der Haftung der Calciumphosphatschichten am Trägerwerkstoff eine abgestufte Resorbierbarkeit mit einer hier sogenannten Wachstumsbremse für den Knochen zu schaffen. Wesentlich ist, daß das Knochenwachstum nach dem Resorbieren der ihm zugewandten äußeren Oberschicht aus leicht resorbierbarem Tricalciumphosphat in einer reinen Calciumphosphatschicht (Bremsschicht) zum Stillstand kommt und jede Löslichkeit auf ein Minimum herabgesetzt ist. Die Quantität der verbleibenden Bremsschicht und der darunter befindlichen Trägerschicht aus nicht resorbierbarem, hoch gesintertem Tricalciumphosphat oder aus nicht resorbierbarem Tetracalciumphosphat dienen dem Knochen in seinem periodischen - gegebenenfalls nach Jahren wiedereinsetzenden - Resorptionsverhalten als Calciumphosphatreservoir verbleibende Brems- und Trägerschicht geben dem Knochengewebe die Möglichkeit einer erneuten Dissoziation und eines Grenzmetamorphismus mit Calcium und Phosphationen. Die unterste, nicht resorbierbare Trägerschicht kann auch als reine Isolierschicht insbesondere auch für den Fall einer aggressiven Resorption im jugendlichen Knochen bezeichnet werden.

Unter hochgesintertem Tricalciumphosphat wird verstanden, daß es bei Temperaturen von über $2100^{\circ}K$ aufgepresst worden ist. Unter einer niedrig gesinterten Calciumphosphatschicht wird hier eine unter etwa $1900^{\circ}K$ aufgepreßte Schicht verstanden.

Einsatzgebiet dieses erfindungsgemäßen Verbundwerkstoffes ist die gesamte zahnärztliche Implantologie, d.h. sämtliche damit herstellbare Formen sowie deren Randgebiete. Dies bedeutet, dass Wurzelstifte, Transfixationsstifte und Knochenschienen damit hergestellt werden können. Außerdem läßt sich dieser Verbundwerkstoff ausdehnen in die Bereiche der gesamten orthopädischen Chirurgie bis hin zu den Knochenschrauben. Der erfindungsgemäße Verbundwerkstoff kann auch auf bekannte Kernstrukturen (Metallkörper für Hüftgelenkprothesen, Knochenschrauben und dergleichen) aufgesintert werden.

Vorteil des erfindungsgemäßen Verbundwerkstoffes ist: Genormte Herstellbarkeit der Formen, grazile Ausformung der Implantatkörper, bioreaktives Verhalten der Implantatkörper-Oberfläche im Knochengewebe, relativ einfache und preiswerte Herstellung der Implantatkörper.

Zum mikrostrukturellen Aufbau des Werkstoffes läßt sich folgendes sagen: der physikalisch-chemische Verbund von Calciumphosphaten und den oben genannten sinterfähigen Metallen ist nachgewiesen. Es gibt eine marmorierte mikrostrukturelle und feinporöse Werkstoffstruktur, die den angeforderten mechanischen Belastungen standhält. Die aufgetragene Calciumphosphatschicht als Oberflächenschicht wird besonders an den Calciumphosphatteilen haften, ist aber ebenso auf die sinterfähigen Metalle aufzupressen und geht mit diesen eine stabile Verbindung ein.

In den beigefügten Zeichnungen sind beispielsweise Knochen-Implantatkörper aus dem erfindungsgemäßen Verbundwerkstoff dargestellt. Es zeigen

Fig. 1 schematisch im Schnitt ein dentales Implantat;

Fig. 2 schematisch im Schnitt eine Knochenschraube;

Fig. 3 schematisch teilweise im Schnitt eine Hüftgelenkprothese;

Fig. 4 schematisch stark vergrößert und im Ausschnitt den Randbereich eines Implantatkörpers;

Fig. 5 schematisch stark vergrößert und im Ausschnitt den Randbereich eines Implantatkörpers anderer Ausführungsform.

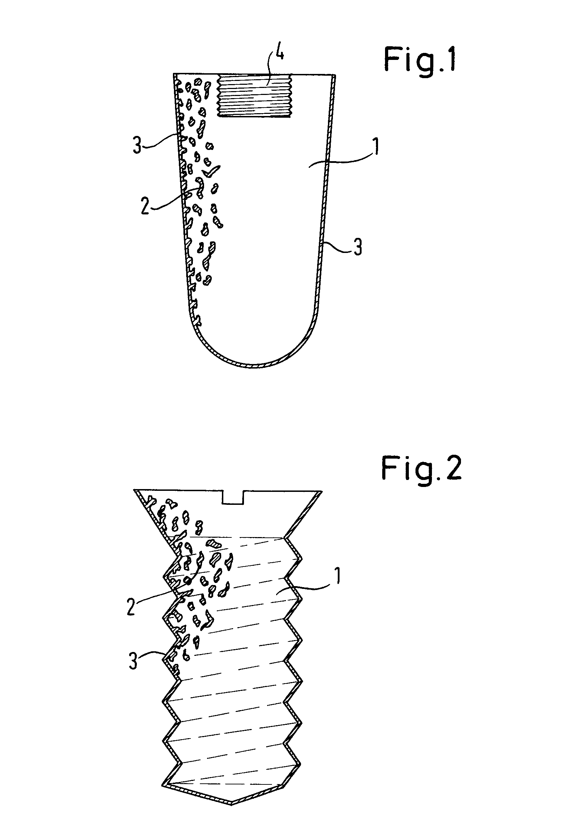

Der dentale Implantatkörper nach Fig. 1 besteht aus Metall-Calciumphosphatkeramik-Sinterverbundwerkstoff 1, wobei die Metallbereiche weiß und die durch Sintern eingelagerten Calciumphosphatbereiche dunkel schraffiert dargestellt sind und mit 2 bezeichnet sind. Der Einfachheit halber sind die Calciumphosphateinschlüsse 2 nur über einen Teilbereich eingezeichnet, obwohl sie sich über den ganzen Verbundwerkstoff 1 erstrecken. Der Implantatkörper ist mit einer Oberflächenschicht 3 aus reiner Calciumphosphatkeramik versehen, deren Dicke in der Zeichnung stark übertrieben dargestellt ist. Entsprechend sind auch die Calciumphosphateinschlüsse 2 übertrieben groß dargestellt, was nicht dem mikroskopischen Bild entspricht. In seinem oberen Bereich weist der Implantatkörper eine Gewindeöffnung 4 zur Aufnahme und Befestigung einer dentalen Suprastruktur wie Zahn, Brücke oder dergleichen auf.

Die in Fig. 2 gezeigte Knochenschraube besteht aus dem gleichen Verbundwerkstoff 1 mit eingelagerten Calciumphosphatbereichen 2 und einer Oberflächenschicht 3. Darstellungsart und Darstellungsgröße entsprechen Fig.1.

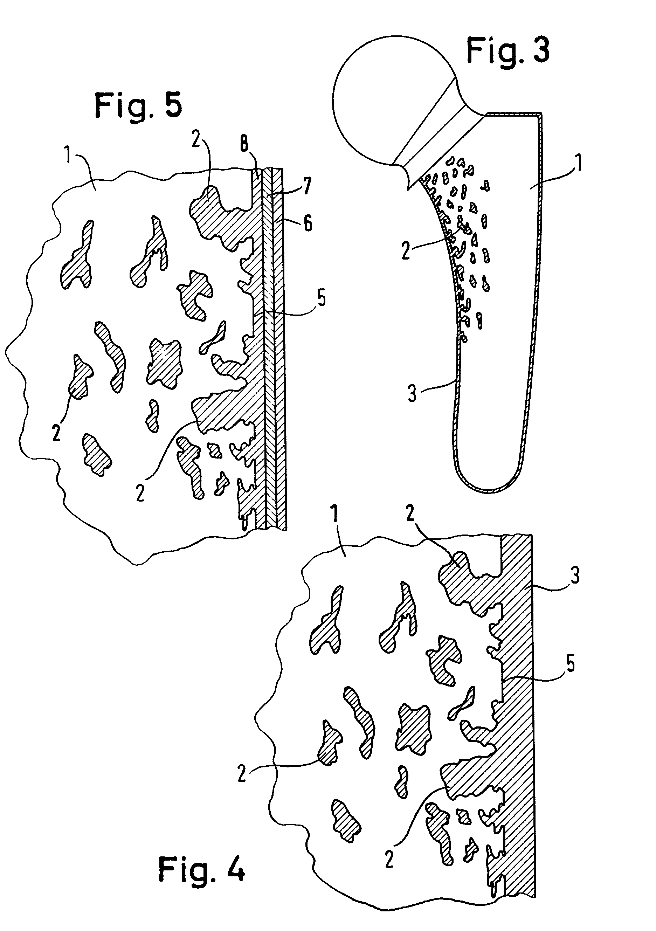

Fig. 3 zeigt schematisch eine Hüftgelenkprothese aus dem erfindungsgemäßen Verbundwerkstoff 1 mit Calciumphosphateinschlüssen 2 und einer Oberflächenschicht 3 aus reinem Calciumphosphat. Die Darstellungsweise entspricht derjenigen in Fig. 1.

In Fig. 4 ist im stark vergrößertem Maßstab dargestellt, wie die Oberflächenschicht 3 aus reinem Calciumphosphat mit dem Verbundwerkstoff 1 durch an der Oberfläche 5 des Werkstoffes 1 freiliegenden Calciumphosphateinschlüsse 2 homogen verankert ist. Zur Verdeutlichung der Größenordnung wird darauf verwiesen, daß die Oberflächenschicht 3 ca. 0,1 bis 0,5 mm dick ist.

Bei der in Fig. 5 dargestellten Ausführungsform besteht die Oberflächenschicht aus reinem Calciumphosphat aus mehreren verschieden hoch gesinterten oder verschieden hoch gepreßten, übereinander gelagerten Calciumphosphat-Schichten, einer äußeren Oberschicht 6, einer mittleren, das Knochenwachstum bremsenden Schicht, sogenannte Bremsschicht 7 und einer inneren Trägerschicht 8.

Patentansprüche

1. Knochenimplantatkörper für Knochen- und Gelenk-, sowie dentale Prothesen, Knochenverbindungsstücke wie Knochenschrauben und -schienen, bestehend aus einem mechanisch stabilen und mit Knochengewebe verträglichen (biokompatiblen) Trägerwerkstoff und darin eingelagerter bioreaktiver Calciumphosphatkeramik (2) insbesondere aus Tri- und Tetracalciumphosphat, dadurch gekennzeichnet, daß der Trägerwerkstoff (1) ein biokompatibles Metall, wie Titan, Gold, Platin, rostfreier Stahl oder ein ähnliches schadlos ohne Bildung von Zwischenreaktionsprodukten mit Calciumphosphatkeramik verbundfähiges Sintermetall ist.

2. Implantatkörper nach Anspruch 1, dadurch gekennzeichnet, daß die Calciumphosphatkeramik (2) im wesentlichen im Bereich der Oberfläche des Metall- oder Sintermetallkörpers (1) angeordnet bzw. angereichert ist.

3. Implantatkörper nach Anspruch 1 oder 2, dadurch gekennzeichnet, daß er zumindest an seinen der Knochenfläche im Implantatraum innerhalb des Knochens zugewandten Seiten eine vollständig aus Calciumphosphat, insbesondere Tricalciumphosphat bestehende Oberflächenschicht (3) aufweist.

4. Implantatkörper nach Anspruch 3, dadurch gekennzeichnet, dass die Schicht (3) 0,1 bis 0,5 mm stark ist.

5. Implantatkörper nach Anspruch 3, dadurch gekennzeichnet, dass die Oberflächenschicht (3) durch degraduiert aufgebrachte Sinterdrücke im Bereich ihrer äußeren Oberfläche resorbierbar ist und mit zunehmender Tiefe zum Implantatkörper (1) hin immer weniger bis nicht resorbierbar ist.

6. Implantatkörper nach Anspruch 3, dadurch gekennzeichnet, daß die Oberflächenschicht aus mehreren verschieden hoch gesinterten oder verschieden hoch gepreßten, übereinander gelagerten Calciumphosphat-Schichten besteht, wobei die äußere Oberschicht (6) aus reinem niedrig gesintertem gut resorbierbarem Tricalciumphosphat, eine mittlere, das Knochenwachstum bremsende Schicht (7) aus hochgesintertem, schwer bis nicht resorbierbaren Tricalciumphosphat und eine innere Trägerschicht (8) aus nicht resorbierbaren, hochgesinterten Calciumphosphat bestehen.

7. Implantatkörper nach Anspruch 6, dadurch gekennzeichnet, daß die innere Trägerschicht (8) aus nicht resorbierbaren Tetracalciumphosphat gegebenenfalls in Mischung mit hochgesintertem Tricalciumphosphat besteht.

8. Verfahren zur Herstellung eines Implantatkörpers nach den Ansprüchen 1 bis 7, dadurch gekennzeichnet, daß ein Pulver des biokompatiblen Metalles mit feindispers bis stückig vorliegender Calciumphosphatkeramik vermischt und durch Sintern unter hohem Druck miteinander verbunden werden.

9. Verfahren nach Anspruch 8, dadurch gekennzeichnet, daß Metallpulver und Calciumphosphatkeramik in etwa gleicher Korngröße und etwa gleichem Volumenverhältnis (Raumerfüllung) miteinander vermischt und gesintert werden.

10. Verfahren nach Anspruch 8 oder 9, dadurch gekennzeichnet, dass die Oberflächenschicht aus reinem Calciumphosphat durch einen oder mehrere weitere Druck-Sintervorgänge auf den Implantatkörper aufgepreßt wird.

11. Verfahren nach Anspruch 10, dadurch gekennzeichnet, daß die Oberflächenschicht aus reinem Calciumphosphat durch aufeinanderfolgende degraduierte Druck-Sintervorgänge aufgepreßt wird und im Bereich ihrer äußeren Oberfläche durch relativ niedrige Sintertemperaturen resorbierbar bleibt und mit zunehmender Tiefe zum Implantatkörper hin durch höhere Sintertemperaturen immer weniger bis nicht resorbierbar aufgepreßt wird.

12. Verfahren nach Anspruch 10, dadurch gekennzeichnet, daß die einzelnen übereinandergelagerten Calciumphosphat-Schichten der Oberflächenschicht durch aufeinanderfolgende Druck-Sintervorgänge aufgepreßt werden, wobei die innere Trägerschicht und die Bremsschicht bei vergleichsweise hohen Sintertemperaturen und/oder Drücken und die äußere Oberschicht bei vergleichsweise niedriger Sintertemperatur aufgepreßt werden.

Fig.1

Fig.2

Fig. 5

Fig. 3

Fig. 4

| Europäisches Patentamt | EUROPÄISCHER RECHERCHENBERICHT | | Nummer der Anmeldung |

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| EX | EP - A - 0 006 544 (BATTELLE-INSTITUT)<br><br>* Patentansprüche 3-14; Abbildung 2 * | 1-12 | A 61 F  1/00<br>A 61 B 17/18<br>A 61 C  8/00<br>A 61 K  6/00 |
| | DE - A - 2 127 843 (ONTARIO RESEARCH)<br><br>* Seite 14, letzter Absatz * | 1 | |
| | DE - A - 2 008 010 (SCHNEIDER) | 3 | RECHERCHIERTE SACHGEBIETE (Int. Cl.³) |
| | DE - A - 2 733 394 (G. RIESS)<br><br>* Patentansprüche 6-9; Abbildung 1 * | 3 | A 61 F  1/00<br>A 61 B 17/18 |
| | FR - A - 2 336 913 (SUMITOMO)<br><br>* Seite 4, letzter Absatz; Patentansprüche 1,13,20; Seite 3, letzter Absatz; Abbildung 2B * | 1,3 | |
| A | DE - A - 2 546 824 (E. LEITZ) | | |
| A | DE - A - 2 620 907 (BATTELLE) | | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 11-11-1980 | PELTRE |

EPA form 1503.1  04.78